# FASCICULE DE BREVET EUROPEEN

(11) **EP 1 040 820 B1**
(45) Date de publication et mention de la délivrance du brevet: **23.06.2004**
(21) Numéro de dépôt: 00400245.7
(22) Date de dépôt: 31.01.2000
(51) Int. Cl.: A61K 7/42

(54) **Compositions cosmétiques antisolaires à base d'un melange synergique de filtres et utilisations**
Kosmetische Sonnenschutzmittel auf Basis einer synergistischen Mischung von Filtern und Verwendungen
Cosmetic sunscreen compositions based on synergic filter mixtures and their uses

(30) Priorité: 12.02.1999 FR 9901730
(43) Date de publication de la demande: 04.10.2000
(73) Titulaire: L'OREAL, 75008 Paris (FR)
(72) Inventeur: Candau, Didier, 91570 Bievres (FR)
(74) Mandataire: Miszputen, Laurent

(56) Documents cités:
- EP-A- 0 685 224
- EP-A- 0 775 698
- EP-A- 0 878 469
- WO-A-98/22447
- WO-A-99/08653
- DE-A- 19 645 317

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins deux filtres particuliers, à savoir d'une part l'acide benzène 1,4-di(3-méthylidène-10-camphosulfonique) et, d'autre part, un dérivé de bis-résorcinyl triazine particulier, ces deux filtres étant présents dans des proportions convenant à l'obtention d'un effet de synergie au niveau des indices de protection conférés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, que la combinaison de deux filtres solaires particuliers et déjà connus en soi dans l'état de l'art, permettait, du fait d'un effet de synergie remarquable, d'obtenir des compositions antisolaires présentant des indices de protection nettement améliorés, et en tous cas largement supérieurs à ceux qui peuvent être obtenus soit avec l'un ou l'autre des filtres utilisé seul.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) l'acide benzène 1,4-di(3-méthylidène-10-camphosulfonique), éventuellement sous forme partiellement ou totalement neutralisée, à titre de premier filtre, et (ii) au moins un dérivé de bis-résorcinyl triazine particulier à titre de second filtre ; lesdits premier et second filtres étant présents dans lesdites compositions dans une proportion produisant une activité synergique au niveau des indices de protection solaire conférés.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

L'acide benzène 1,4-di(3-méthylidène-10-camphosulfonique) et ses différents sels, décrits notamment dans les demandes de brevets FR-A-2528420 et FR-A-2639347, sont des filtres déjà connus en soi (filtres dits à large bande) capables en effet d'absorber les rayons ultraviolets de longueur d'ondes comprises entre 280 et 400 nm, avec des maxima d'absorption compris entre 320 et 400 nm, en particulier aux alentours de 345 nm.

Ces filtres répondent à la formule générale (1) suivante : 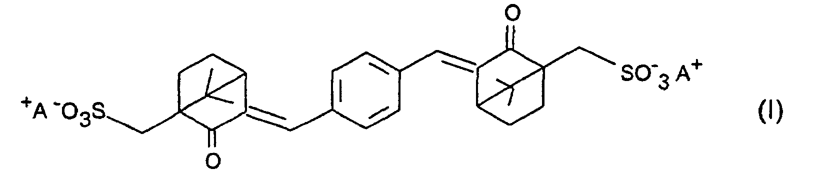 dans laquelle A désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺. Il est bien entendu que les composés de formule (I) ci-dessus peuvent donner lieu à l'isomère "cis-trans" autour d'une ou plusieurs double(s) liaison(s) et que tous les isomères rentrent dans le cadre de la présente invention.

L'acide benzène 1,4-di(3-méthylidène-10-camphosulfonique) et ou l'un de ses différents sels est généralement présent dans les compositions filtrantes selon l'invention à une concentration totale allant de 0,1 à 15 % en poids environ, et de préférence de 0,2 à 10 % en poids environ, par rapport au poids total de la composition.

Les dérivés de bis-résorcinyl triazine, conformes à la présente invention répondent à la formule (II) suivante : 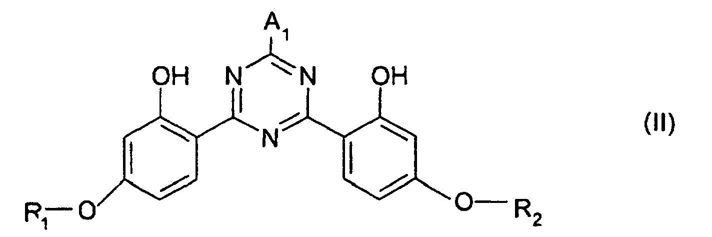 dans laquelle :
(i) les radicaux R₁ et R₂, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ;
(ii) les radicaux R₁ et R₂, identiques ou différents, peuvent encore désigner un reste de formule (1) suivante : 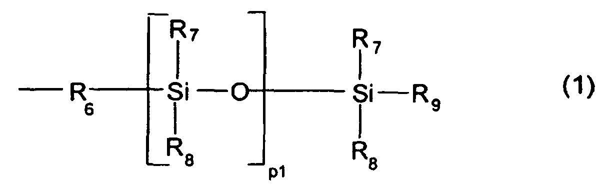
dans laquelle :
- R₆ désigne une liaison covalente ; un radical alkyle linéaire ou ramifié en C₁-C₄ ou bien un reste de formule -Cₘ₁H₂ₘ₁-O- où m₁ est un nombre de 1 à 4 ;
- p₁ est un nombre de 0 à 5 ;
- les radicaux R₇, R₈ et R₉, identiques ou différents, désignent un radical alkyle en C₁-C₁₈ ; un radical alcoxy en C₁-C₁₈ ou un reste de formule: 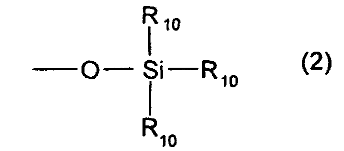 où R₁₀ est un radical alkyle en C₁-C₅ ;
- A₁ désigne un reste répondant à l'une des formules suivantes : 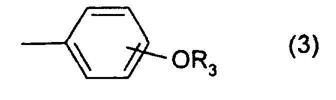 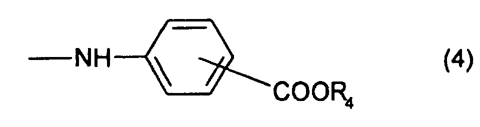 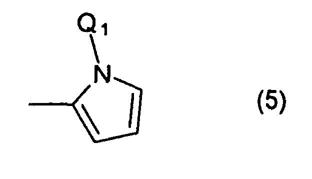 dans lesquelles :
   - R₃ désigne un atome d'hydrogène, un radical alkyle en C₁-C₁₀, un radical de formule : -(CH₂CHR₅-O)ₙ₁R₄ où n₁ est un nombre de 1 à 16, ou bien un reste de structure -CH₂-CH-(OH)-CH₂OT₁ avec T₁ ayant la même signification indiquée ci-dessus.
   - R₄ désigne hydrogène, un cation métallique M, un radical alkyle en C1-C5 ou un reste de formule -(CH₂)m₂-OT₁ où m₂ est un nombre de 1 à 4 et T₁ a la même signification indiquée ci-dessus.
   - Q₁ est un radical alkyle en C₁-C₁₈.

Dans les formules (II) et (1) à (5) décrites ci-dessus :
- les radicaux alkyle sont linéaires ou ramifiés et peuvent être choisis par exemple parmi méthyle, éthyle, n-propyle, isopropyle, n-butyle, sec.butyle, tert.butyle, amyle, isoamyle, tert.amyle, heptyle, octyle, isooctyle, nonyle, décyle, undécyle, dodécyle, tétradécyle, pentadécyle, hexadécyle, heptadécyle ou octadécyle ;
- les radicaux alcényle peuvent être choisis par exemple parmi allyle, méthallyle, isopropènyle, 2-butènyle, 3-butènyle, isobutènyle, n-penta-2,4-diènyle, 3-méthyl-but-2-enyle, n-oct-2-enyle, n-dodec-2-enyle, iso-dodecenyle, n-octadec-4-enyle ;
- les radicaux alcoxy sont linéaires ou ramifiés et peuvent être choisis par exemple parmi méthoxy, éthoxy, n-propoxy, isopropoxy, n-butoxy, sec.-butoxy, tert.-butoxy, amyloxy, isoamyloxy ou tert.amyloxy ;
- les radicaux mono ou dialkylamino en C₁-C₅ peuvent être choisis par exemple parmi méthylamino, éthylamino, propylamino, n-butylamino, sec.butylamino, tert. butylamino, pentylamino, diméthylamino, diéthylamino, dibutylamino ou méthyléthylamino.
- les cations métalliques sont des cations alcalins, alcalino-terreux ou métalliques choisis par exemple parmi lithium, potassium, sodium, calcium, magnésium, cuivre et zinc.

Les dérivés de bis-résorcinyl triazine de formule (II) de l'invention sont des filtres déjà connus en soi. Ils sont décrits et préparés selon les synthèses indiquées dans les demandes de brevet EP-A-0775 698.

A titre d'exemples de composés de formule (II) utilisables, on peut citer :
- la 2,4-bis {[4-(2-éthyl-hexyloxy)-2-hydroxy]-phényl}-6-(4-méthoxy-phenyl)-1,3,5-triazine ;
- la 2,4-bis{[4-(3-(2-propyloxy)-2-hydroxy-propyloxy)-2-hydroxy]-phényl}-6-(4-méthoxyphényl)-1,3,5-triazine ;
- la 2,4-bis {[4-(2-éthyl-hexyloxy)-2-hydroxy]-phényl}-6-[4-(2-méthoxyéthyl-carboxyl)-phénylamino]-1,3,5-triazine ;
- la 2,4-bis {[4-tris(triméthylsiloxy-silylpropyloxy)-2-hydroxy]-phényl}-6-(4-méthoxyphényl)-1,3,5-triazine ;
- la 2,4-bis {[4-(2"-méthylpropenyloxy)-2-hydroxy]-phényl}-6-(4-méthoxyphényl)-1,3,5-triazine ;
- la 2,4-bis {[4-(1',1',1',3',5',5',5'-heptaméthyltrisiloxy-2"-méthylpropyloxy)-2-hydroxy]-phényl}-6-(4-méthoxyphényl)-1,3,5-triazine.
- la 2,4-bis{[4-(3-(2-propyloxy)-2-hydroxy-propyloxy]-2-hydroxy]-phényl}-6-[(4-éthylcarboxyl)-phénylamino]-1,3,5-triazine ;
- la 2,4-bis {[4-(2-éthyl-hexyloxy)-2-hydroxy]-phényl}-6-(1-méthylpyrrol-2-yl)-1,3,5-triazine.

Les composés dérivés de bis-résorcinyl triazine plus particulièrement préférés selon l'invention sont choisis dans le groupe constitué par :
- la 2,4-bis {[4-(2-éthyl-hexyloxy)-2-hydroxy]-phényl}-6-(4-méthoxy-phényl)-1,3,5-triazine ;
- la 2,4-bis {[4-tris(triméthylsiloxy-silylpropyloxy)-2-hydroxy]-phényl}-6-(4-méthoxyphényl)-1,3,5-triazine ;
- la 2,4-bis {[4-(1',1',1',3',5',5',5'-heptaméthyltrisiloxy-2"-méthylpropyloxy)-2-hydroxy]-phényl}-6-(4-méthoxyphényl)-1,3,5-triazine.

Le ou les filtres organiques du type dérivé de bis-résorcinyl triazine de formule (II) sont généralement présents dans les compositions filtrantes selon l'invention à une concentration totale comprise entre 0,1 et 15 % en poids environ, et de préférence entre 0,2 et 10 % en poids environ, par rapport au poids total de la composition.

Comme indiqué précédemment, selon une caractéristique essentielle de la présente invention, il convient que ces deux composés soient tous deux présents dans la composition finale dans une proportion respective telle qu'un effet de synergie au niveau de l'indice de protection conféré par l'association résultante, soit obtenu de manière notable, substantielle et significative.

En outre, et d'une manière générale, on notera que les concentrations et rapports en composés de formule (I) et composés de formule (II) tels que définis précédemment sont choisis de manière telle que l'indice de protection solaire de la composition finale soit de préférence d'au moins 2.

Enfin, toujours selon un mode préféré de réalisation de la présente invention, le support cosmétiquement acceptable dans lequel se trouvent contenus les différents filtres organiques de formule (I) et de formule (II) tels que définis précédemment est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques ; les dérivés salicyliques, les dérivés du camphre ; les dérivés de triazine autres que ceux définis précédemment tels que ceux décrits dans les demandes de brevet EP863145, EP517104, EP570838 et EP796851 ; les dérivés de la benzophénone ; les dérivés du dibenzoylméthane ; les dérivés de β,β'-diphénylacrylate, les dérivés de benzimidazole ; les dérivés de l'acide p-aminobenzoïque ; les polymères filtres et silicones filtres tels que ceux décrits dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide urocanique,
l'acide α-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
la 2,4,6-tris-[ p-(2'-éthylhexyl-1'-oxycarbonyl)anilino]-1,3,5-triazine,
2-[p-(tertiobutylamido)anilino]-4,6-bis-[(p-(2'-éthylhexyl-1'-oxycarbonyl)anilino]-1,3,5-triazine,
l'acide 1,4-bis-benzimidazolyl-phènylèn-3,3',5,5'-tétrasulfonique et ses sels,
le polymère de N-(2 et 4)-[(2-oxobom-3-ylidèn)méthyl] benzyl]-acrylamide,
le drométrizole trisiloxane (nom INCl),
les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly- -oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier les indices de protections solaires, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de poudre, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et peut constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés. Les quantités indiquées sont exprimées en % en poids.

### EXEMPLES

| **COMPOSITION** | **EX 1** |
|---|---|
| Mélange d'alcool cétylstéarylique et d'alcool cétylstéarylique oxyéthyléné (33 OE) 80/20 (SINNOWAX AO -HENKEL) | 7 |
| Mélange de mono et distéarate de glycérol (CERASYNT SD-V ISP) | 2 |
| Alcool cétylique | 1.5 |
| Polydiméthyl siloxane (DOW CORNING 200 FLUID -DOW CORNING) | 1 |
| Benzoate d'alcools en C12/C15 (WITCONOL TN -WITCO) | 15 |
| 2,4-bis-{[4-(2-éthyl-hexyloxy)-2-hydroxy]-phényl}-6-(4-methoxyphenyl)-1,3,5-triazine | 2 |
| Glycérine | 15 |
| Acide benzène 1,4-di(3-méthylidène-10-camphosulfonique) (MEXORYL SX-CHIMEX) | 2 |
| Triéthanolamine | qs pH 7 |
| Conservateurs | qs |
| Eau déminéralisée qsp | 100 g |

| **COMPOSITION** | **EX 2** |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2.5g |
| Poly diméthylsiloxane (DOW CORNING 200 FLUID - DOW CORNING) | 0.5g |
| Benzoate d'alcools en C₁₂/C₁₅ (WITCONOL TN -WITCO) | 20g |
| Triéthanolamine | 0.5g |
| 2,4-Bis-{[4-tris(trimethylsiloxy-silylpropyloxy)-2-hydroxy]-phenyl}-6-(4-methoxyphenyl)-1,3,5-triazine | 2.5g |
| Propylène glycol | 4g |
| Glycérine | 4g |
| Acide benzène 1,4-di(3-méthylidêne-10-camphosulfonique) (MEXORYL SX-CHIMEX) | 2.5g |
| Phosphate d'alcool hexadécylique,sel de potassium (AMPHISOL K - HOFFMAN LAROCHE) | 0.5g |
| Triéthanolamine | qs pH :7 |
| Acide polyacrylique (SYNTHALEN K - 3V) | 0.3g |
| Hydroxypropyl méthyl cellulose (METHOCEL F4M -DOW CHEMICAL) | 0.1g |
| Conservateurs | qs |
| Eau démineralisée qsp | 100 g |

| **COMPOSITION** | **EX 3** |
|---|---|
| Mélange d'alcool cétylstéarylique et d'alcool cétylatéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en C₁₂/C₁₅ (WITCONOL TN -WITCO) | 15g |
| 2,4-Bis-{(4-(1',1',1',3',5',5',5'-Heptaméthyltrisiloxy-2''-méthyl-propyloxy)-2-hydroxy]-phényl}-6-(4-méthoxyphenyl)-1,3,5-triazine | 2g |
| Glycérine | 15g |
| Acide benzène 1,4-di(3-méthylidène-10-camphosulfonique) (MEXORYL SX-CHIMEX) | 2g |
| Triéthanolamine | qs pH 7 |
| Conservateurs | qs |
| Eau déminéralisée qsp | 100 g |

## Revendications

1. Composition cosmétique à usage topique, en particulier pour la photoprotection de la peau et/ou des cheveux, **caractérisée par le fait qu'**elle comprend, dans un support cosmétiquement acceptable :
(i) l'acide benzène 1,4-di(3-méthylidène-10-camphosulfonique), éventuellement sous forme partiellement ou totalement neutralisée, à titre de premier filtre,
(ii) et à titre de second filtre, au moins un dérivé de bis-résorcinyl triazine répondant à la formule (II) suivante : 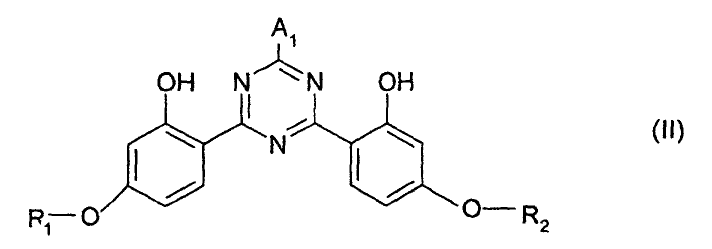
dans laquelle :
(i) les radicaux R₁ et R₂, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ;
(ii) les radicaux R₁ et R₂, identiques ou différents, peuvent encore désigner un reste de formule (1) suivante : 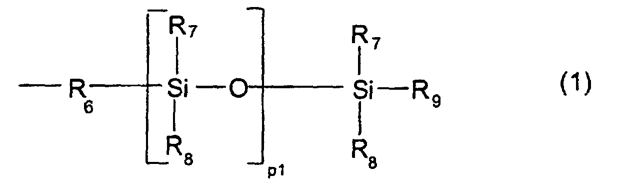
dans laquelle :
- R₆ désigne une liaison covalente ; un radical alkyle linéaire ou ramifié en C₁-C₄ ou bien un reste de formule -Cₘ₁H₂ₘ₁-O- où m₁ est un nombre de 1 à 4 ;
- p₁ est un nombre de 0 à 5 ;
- les radicaux R₇, R₈ et R₉, identiques ou différents, désignent un radical alkyle en C₁-C₁₈ ; un radical alcoxy en C₁-C₁₈ ou un reste de formule : 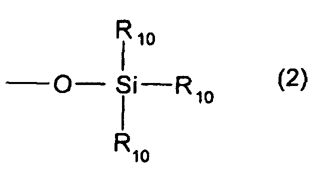 où R₁₀ est un radical alkyle en C₁-C₅ ;
- A₁ désigne un reste répondant à l'une des formules suivantes : 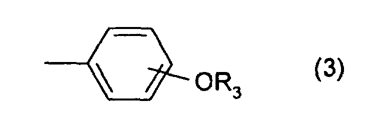 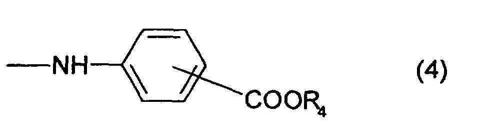 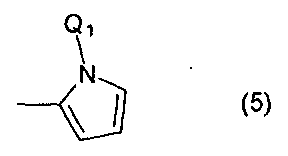
dans lesquelles :
- R₃ désigne un atome d'hydrogène, un radical alkyle en C₁-C₁₀, un radical de formule : -(CH₂CHR₅-O)ₙ₁R₄ où n₁ est un nombre de 1 à 16, ou bien un reste de structure -CH₂-CH-(OH)-CH₂OT₁ avec T₁ ayant la même signification indiquée ci-dessus.
- R₄ désigne hydrogène, un cation métallique M, un radical alkyle en C1-C5 ou un reste de formule -(CH₂)m₂-OT₁ où m₂ est un nombre de 1 à 4 et T₁ a la même signification indiquée ci-dessus.
- Q₁ est un radical alkyle en C₁-C₁₈ ; lesdits premier et second filtres étant présents dans une proportion produisant une activité synergique au niveau des indices de protection solaire conférés.

2. Composition selon la revendication 1, où le composé de formule (II) est choisi dans le groupe constitué par :
- la 2,4-bis {[4-(2-éthyl-hexyloxy)-2-hydroxy]-phényl}-6-(4-méthoxy-phenyl)-1,3,5-triazine ;
- la 2,4-bis{[4-(3-(2-propyloxy)-2-hydroxy-propyloxy)-2-hydroxy]-phényl}-6-(4-méthoxyphényl)-1,3,5-triazine ;
- la 2,4-bis {[4-(2-éthyl-hexyloxy)-2-hydroxy]-phényl}-6-[4-(2-méthoxyéthyl-carboxyl)-phénylamino]-1,3,5-triazine ;
- la 2,4-bis {[4-tris(triméthylsiloxy-silylpropyloxy)-2-hydroxy]-phényl}-6-(4-méthoxyphényl)-1,3,5-triazine ;
- la 2,4-bis {[4-(2"-méthylpropenyloxy)-2-hydroxy]-phényl}-6-(4-méthoxyphényl)-1,3,5-triazine ;
- la 2,4-bis {[4-(1',1',1',3',5',5',5'-heptaméthyltrisiloxy-2"-méthylpropyloxy)-2-hydroxy]-phényl}-6-(4-méthoxyphényl)-1,3,5-triazine.
- la 2,4-bis{[4-(3-(2-propyloxy)-2-hydroxy-propyloxy]-2-hydroxy]-phényl}-6-[(4-éthylcarboxyl)-phénylamino]-1,3,5-triazine ;
- la 2,4-bis {[4-(2-éthyl-hexyloxy)-2-hydroxy]-phényl}-6-(1-méthylpyrrol-2-yl)-1,3,5-triazine.

3. Composition selon l'une quelconque des revendications 1 et 2, **caractérisée par le fait que** le composé de formule (II) est choisi dans le groupe constitué par :
- la 2,4-bis {[4-(2-éthyl-hexyloxy)-2-hydroxy]-phényl}-6-(4-méthoxy-phenyl)-1,3,5-triazine ;
- la 2,4-bis {[4-tris(triméthylsiloxy-silylpropyloxy)-2-hydroxy]-phényl}-6-(4-méthoxyphenyl)-1,3,5-triazine ;
- la 2,4-bis {[4-(1',1',1',3',5',5',5'-heptaméthyltrisiloxy-2"-méthylpropyloxy)-2-hydroxy]-phényl}-6-(4-méthoxyphényl)-1,3,5-triazine.

4. Composition selon l'une quelconque des revendications 1 à 3, **caractérisée par le fait que** la concentration en filtre du type dérivé de bis-résorcyl triazine est comprise entre 0,1 et 15 % en poids par rapport au poids total de la composition.

5. Composition selon la revendication 4, **caractérisée par le fait que** ladite concentration est comprise entre 0,2 et 10 % en poids par rapport au poids total de la composition.

6. Composition selon l'une quelconque des revendications 1 à 5, **caractérisée par le fait que** l'acide benzène 1,4-di(3-méthylidène-10-camphosulfonique), et ses sels répond à la formule (I) suivante : 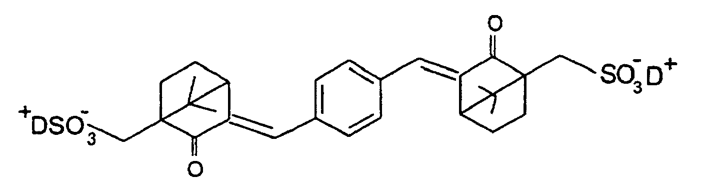 dans laquelle D désigne un atome d'hydrogène, un métal alcalin ou encore un radical NH(R₂₅)₃⁺ dans lequel les radicaux R₂₅, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺.

7. Composition selon l'une quelconque des 1 à 6, **caractérisée par le fait que** l'acide benzène 1,4-di(3-méthylidène-10-camphosulfonique), ou l'un de ses sels est généralement présent à une concentration totale comprise entre 0,1 et 15 % en poids environ par rapport au poids total de la composition.

8. Composition selon la revendication 7 où la concentration est comprise entre 0,2 et 10 % en poids environ, par rapport au poids total de la composition

9. Composition selon l'une quelconque des revendications 1 à 8, **caractérisée par le fait que** ledit support cosmétiquement acceptable se présente sous la forme d'une émulsion de type huile-dans-eau.

10. Composition selon l'une quelconque des revendications 1 à 9, **caractérisée par le fait qu'**elle comprend en outre un ou plusieurs filtres organiques complémentaires actifs dans l'UV-A et/ou UV-B, hydrophiles ou lipophiles, différents desdits premier et deuxième filtres.

11. Composition selon la revendication 10, **caractérisée par le fait que** lesdits filtres organiques complémentaires sont choisis parmi les dérivés de cinnamate, dérivés salicyliques, les dérivés du camphre, les dérivés de benzimidazole, les dérivés de β,β-diphénylacrylate, les dérivés de triazine autres que ceux définis dans les revendications précédentes, les dérivés de l'acide p-aminobenzoïque, les polymères filtres et silicones filtres.

12. Composition selon l'une quelconque des revendications1 à 11, **caractérisée par le fait qu'**elle comprend en outre, à titre d'agents photoprotecteurs UV complémentaires, des pigments ou des nanopigments d'oxydes métalliques, enrobés ou non.

13. Composition selon la revendication 12, **caractérisée par le fait que** lesdits pigments ou nanopigments sont choisis parmi les oxydes de titane, de zinc, de fer, de zirconium, de cérium et leurs mélanges, enrobés ou non.

14. Composition selon l'une quelconque des revendications 1 à 13, **caractérisée par le fait qu'**elle comprend en outre au moins un agent de bronzage et/ou de brunissage artificiel de la peau.

15. Composition selon l'une quelconque des revendications 1 à 14, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

16. Composition selon l'une quelconque des revendications 1 à 15, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

17. Composition selon l'une quelconque des revendications 1 à 16, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

18. Composition selon l'une quelconque des revendications 1 à 16, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

19. Utilisation de la composition définie à l'une quelconque des 1 à 16 précédentes pour la fabrication de compositions cosmétiques pour la protection de la peau et/ou des cheveux contre le rayonnement ultraviolet, en particulier le rayonnement solaire.

20. Procédé de traitement cosmétique pour protéger la peau et/ou les cheveux contre le rayonnement ultraviolet, en particulier le rayonnement solaire, **caractérisé en ce qu'**il consiste à appliquer sur ceux-ci une quantité efficace d'une composition telle que définie à l'une quelconque des revendications 1 à 18.

## Patentansprüche

1. Kosmetische Zusammensetzung zur topischen Anwendung, insbesondere zum Lichtschutz der Haut und/oder der Haare, **dadurch gekennzeichnet, dass** sie in einem kosmetisch akzeptablen Träger enthält:
(i) als erstes Filter die Benzol-1,4-di(3-methyliden-10-camphersulfonsäure) gegebenenfalls in ganz oder teilweise neutralisierter Form, und
(ii) als zweites Filter mindestens ein Bis-resorcinyl-triazinderivat der folgenden Formel (II): 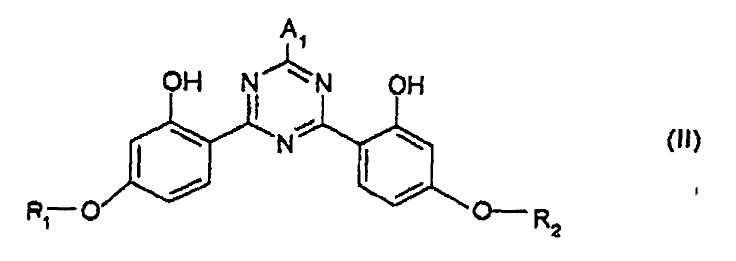 wobei in der Formel bedeuten:
(i) die Gruppen R₁ und R₂, die gleich oder verschieden sind, bedeuten eine C₃₋₁₈-Alkylgruppe, eine C₂₋₁₈-Alkenylgruppe oder einen Rest der Formel -CH₂-CH(OH)-CH₂-OT₁, wobei T₁ ein Wasserstoffatom oder eine C₁₋₈-Alkylgruppe bedeutet;
(ii) die Gruppen R₁ und R₂, die gleich oder verschieden sind, können auch eine Gruppe der folgenden Formel (1) bedeuten: 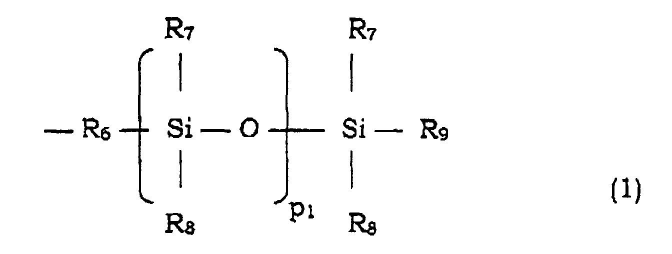 worin bedeuten:
- R₆ eine kovalente Bindung, eine geradkettige oder verzweigte C₁₋₄-Alkylgruppe oder einen Rest der Formel -Cₘ₁H₂ₘ₁-O-, wobei m₁ eine Zahl von 1 bis 4 ist,
- p₁ eine Zahl von 0 bis 5,
- die Gruppen R₇, R₈ und R₉, die gleich oder verschieden sind, eine C₁₋₁₈-Alkylgruppe, eine C₁₋₁₈-Alkoxygruppe oder eine Gruppe der Formel: 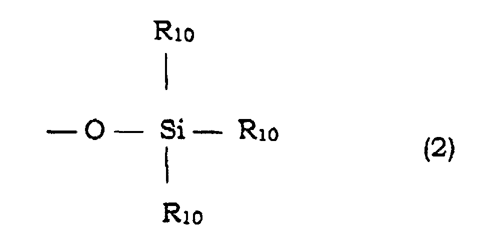 wobei R₁₀ eine C₁₋₅-Alkylgruppe bedeutet,
- A₁ einen Rest, der einer der folgenden Formel entspricht: 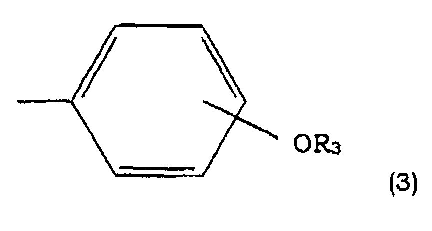 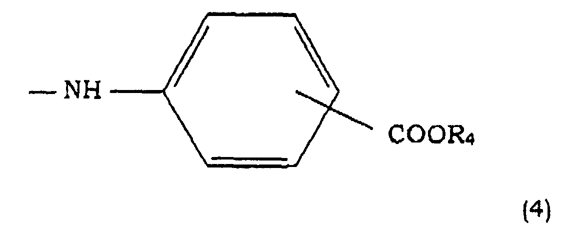 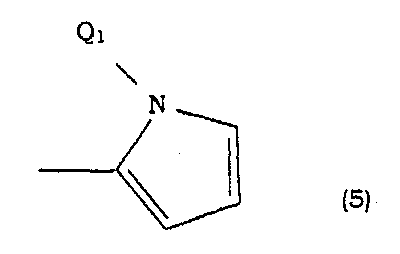
worin bedeuten:
- R₃ ein Wasserstoffatom, eine C₁₋₁₀-Alkylgruppe, eine Gruppe der Formel -(CH₂CHR₅-O)ₙ₁R₄, worin n₁ eine Zahl von 1 bis 16 bedeutet, oder einen Rest der Formel -CH₂-CH(OH)-CH₂-OT₁, wobei T₁ die oben angegebenen Bedeutungen aufweist;
- R₄ ein Wasserstoffatom, ein Metallkation M, eine C₁₋₅-Alkylgruppe oder eine Gruppe der Formel -(CH₂)ₘ₂OT₁, worin m₂ eine Zahl von 1 bis 4 bedeutet und T₁ die oben angegebenen Bedeutungen aufweist;
- Q₁ eine C₁₋₁₈-Alkylgruppe,
wobei das erste und das zweite Filter in Mengenanteilen enthalten sind, die hinsichtlich der erzielten Lichtschutzfaktoren zu einer synergistischen Wirkung führen.

2. Zusammensetzung nach Anspruch 1, wobei die Verbindung der Formel (II) unter den folgenden Verbindungen ausgewählt ist:
- 2,4-Bis{[4-(2-ethyl-hexyloxy)-2-hydroxy]-phenyl}-6-(4-methoxyphenyl)-1,3,5-triazin;
- 2,4-Bis{[4-(3-(2-propyloxy)-2-hydroxy-propyloxy)-2-hydroxy]-phenyl}-6-(4-methoxyphenyl)-1,3,5-triazin;
- 2,4-Bis{[4-(2-ethyl-hexyloxy)-2-hydroxy]-phenyl}-6-[4-(2-methoxyethyl-carboxy)-phenylamino)-1,3,5-triazin;
- 2,4-Bis{[4-tris(trimethylsiloxy-silylpropyloxy)-2-hydroxy]-phenyl}-6-(4-methoxyphenyl)-1,3,5-triazin;
- 2,4-Bis{[4-(2"-methylpropenyloxy)-2-hydroxy]-phenyl}-6-(4-methoxyphenyl)-1,3,5-triazin;
- 2,4-Bis{[4-(1',1',1',3',5',5',5'-heptamethyltrisiloxy-2"-methylpropyloxy)-2-hydroxy]-phenyl}-6-(4-methoxyphenyl)-1,3,5-triazin;
- 2,4-Bis{[4-(3-(2-propyloxy)-2-hydroxy-propyloxy]-2-hydroxy]-phenyl}-6-([4-ethylcarboxy)-phenylamino)-1,3,5-triazin;
- 2,4-Bis{[4-(2-ethyl-hexyloxy)-2-hydroxy]-phenyl}-6-(1-methylpyrrol-2-yl)-1,3,5-triazin.

3. Zusammensetzung nach einem der Ansprüche 1 und 2, **dadurch gekennzeichnet, dass** die Verbindung der Formel (II) unter:
- 2,4-Bis{[4-(2-ethyl-hexyloxy)-2-hydroxy]-phenyl}-6-(4-methoxyphenyl)-1,3,5-triazin;
- 2,4-Bis{[4-tris(trimethylsiloxy-silylpropyloxy)-2-hydroxy]-phenyl}-6-(4-methoxyphenyl)-1,3,5-triazin; und
- 2,4-Bis{[4-(1',1',1',3',5',5',5'-heptamethyltrisiloxy-2"-methylpropyloxy)-2-hydroxy]-phenyl}-6-(4-methoxyphenyl)-1,3,5-triazin ausgewählt ist.

4. Zusammensetzung nach einem der Ansprüche 1 bis 3, **dadurch gekennzeichnet, dass** die Konzentration des Filters vom Typ der Bis-resorcinyl-triazinderivate im Bereich von 0,1 bis 15 Gew.-%, bezogen auf das Gesamtgewicht der Zusammensetzung, liegt.

5. Zusammensetzung nach Anspruch 4, **dadurch gekennzeichnet, dass** die Konzentration im Bereich von 0,2 bis 10 Gew.-%, bezogen auf das Gesamtgewicht der Zusammensetzung, liegt.

6. Zusammensetzung nach einem der Ansprüche 1 bis 5, **dadurch gekennzeichnet, dass** die Benzol-1,4-di(3-methyliden-10-camphersulfonsäure) oder eines ihrer Salze der folgenden Formel entspricht: 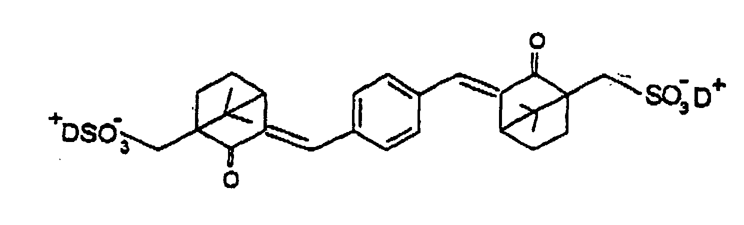 wobei D ein Wasserstoffatom, ein Alkalimetall oder eine Gruppe NH(R₂₅)₃⁺ bedeutet, in der die Gruppen R₂₅, die gleich oder verschieden sein können, ein Wasserstoffatom, eine Alkylgruppe oder eine Hydroxyalkylgruppe mit 1 bis 4 Kohlenstoffatomen oder eine Gruppierung Mⁿ⁺/n bedeuten, wobei Mⁿ⁺ ein mehrwertiges Metallkation ist und n 2 oder 3 oder 4 bedeutet und wobei Mⁿ⁺ vorzugsweise ein Metallkation ist, das unter Ca²⁺, Zn²⁺, Mg²⁺, Ba²⁺, Al³⁺ und Zr⁴⁺ ausgewählt ist.

7. Zusammensetzung nach einem der Ansprüche 1 bis 6, **dadurch gekennzeichnet, dass** die Benzol-1,4-di(3-methyliden-10-camphersulfonsäure) oder eines ihrer Salze im Allgemeinen in einer Gesamtkonzentration von etwa 0,1 bis 15 Gew.-%, bezogen auf das Gesamtgewicht der Zusammensetzung, enthalten ist.

8. Zusammensetzung nach Anspruch 7, wobei die Konzentration im Bereich von etwa 0,2 bis 10 Gew.-%, bezogen auf das Gesamtgewicht der Zusammensetzung, liegt.

9. Zusammensetzung nach einem der Ansprüche 1 bis 8, **dadurch gekennzeichnet, dass** der kosmetisch akzeptable Träger als Emulsion von Öl-in-Wasser-Typ vorliegt.

10. Zusammensetzung nach einem der Ansprüche 1 bis 9, **dadurch gekennzeichnet, dass** sie ferner ein oder mehrere, ergänzende, hydrophile oder lipophile organische Filter, die im UV-A-Bereich und/ oder UV-B-Bereich wirksam sind und die von dem genannten ersten und zweiten Filter verschieden sind, enthält.

11. Zusammensetzung nach Anspruch 10, **dadurch gekennzeichnet, dass** die ergänzenden organischen Filter unter den Cinnamatderivaten, Salicylsäurederivaten, Campherderivaten, Benzimidazolderivaten, β,β-Diphenylacrylatderivaten, Triazinderivaten, die von den in den vorhergehenden Ansprüchen definierten Triazinderivaten verschieden sind, p-Aminobenzoesäurederivaten, polymeren Filtern und Siliconfiltern ausgewählt werden.

12. Zusammensetzung nach einem der Ansprüche 1 bis 11, **dadurch gekennzeichnet, dass** sie als ergänzende UV-Lichtschutzmittel ferner gegebenenfalls umhüllte Pigmente oder Nanopigmente von Metalloxiden enthält.

13. Zusammensetzung nach Anspruch 12, **dadurch gekennzeichnet, dass** die Pigmente oder Nanopigmente unter den gegebenenfalls umhüllten Oxiden von Titan, Zink, Eisen, Zirconium oder Cer und deren Gemischen ausgewählt sind.

14. Zusammensetzung nach einem der Ansprüche 1 bis 13, **dadurch gekennzeichnet, dass** sie außerdem mindestens ein Bräunungsmittel und/oder Mittel zur künstlichen Braunfärbung der Haut enthält.

15. Zusammensetzung nach einem der Ansprüche 1 bis 14, **dadurch gekennzeichnet, dass** sie ferner mindestens einen Zusatzstoff enthält, der unter den Fettsubstanzen, organischen Lösungsmitteln, Verdickungsmitteln, beruhigenden Stoffen, Antioxidantien, Trübungsmitteln, Stabilisatoren, Emollientien, Hydroxysäuren, Schaumverhütungsmitteln, Hydratisierungsmitteln, Vitaminen, Parfums, Konservierungsmitteln, grenzflächenaktiven Stoffen, Füllstoffen, Maskierungsmitteln, Polymeren, Treibmitteln, Alkalisierungsmitteln, Ansäuerungsmitteln und Farbmitteln ausgewählt ist.

16. Zusammensetzung nach einem der Ansprüche 1 bis 15, **dadurch gekennzeichnet, dass** es sich um Zusammensetzungen, die die menschliche Epidermis schützen, oder Sonnenschutzmittel handelt und diese als nichtionische Vesikeldispersionen, Emulsionen, insbesondere Öl-in-Wasser-Emulsionen, Cremes, Milche, Gele, Gelcremes, Suspensionen, Dispersionen, Puder, feste Stifte, Schäume und Sprays vorliegen.

17. Zusammensetzung nach einem der Ansprüche 1 bis 16, **dadurch gekennzeichnet, dass** es sich um Zusammensetzungen zum Schminken der Wimpern, Augenbrauen oder der Haut handelt und dass sie in fester oder pastöser, wasserfreier oder wässriger Form, als Emulsionen, Suspensionen oder Dispersionen vorliegen.

18. Zusammensetzung nach einem der Ansprüche 1 bis 16, **dadurch gekennzeichnet, dass** es sich um Zusammensetzungen zum Schutz der Haare gegen UV-Strahlung handelt und dass sie als Haarwaschmittel, Lotionen, Gele, Emulsionen oder nichtionische Vesikeldispersionen vorliegen.

19. Verwendung der Zusammensetzung nach einem der Ansprüche 1 bis 16 für die Herstellung von kosmetischen Produkten zum Schutz der Haut und/oder der Haare gegen UV-Strahlung und insbesondere gegen Sonnenlicht.

20. Verfahren zur kosmetischen Behandlung zum Schutz der Haut und/oder der Haare gegen UV-Strahlung, insbesondere Sonnenlicht, **dadurch gekennzeichnet, dass** auf diese eine wirksame Menge einer Zusammensetzung nach einem der Ansprüche 1 bis 18 aufgetragen wird.

## Claims

1. Cosmetic composition for topical use, in particular for the photoprotection of the skin and/or hair, **characterized in that** it comprises, in a cosmetically acceptable vehicle:
(i) benzene-1,4-di(3-methylidene-10-camphorsulphonic acid), optionally in partially or completely neutralized form, as first screening agent,
(ii) and, as second screening agent, at least one bisresorcinyltriazine derivative corresponding to the following formula (II) : 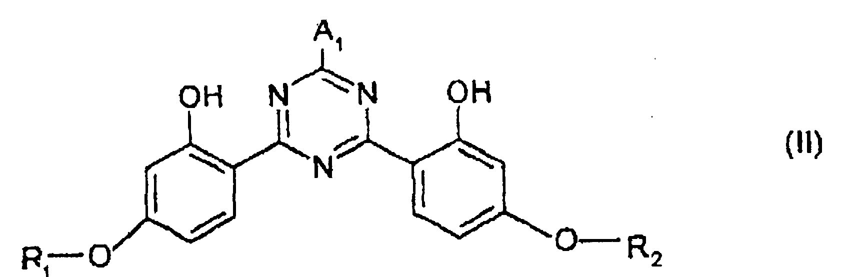 in which:
(i) the R₁ and R₂ radicals, which are identical or different, denote a C₃-C₁₈ alkyl radical, a C₂-C₁₈ alkenyl radical or a residue of formula -CH₂-CH(OH)-CH₂-OT₁ where T₁ is a hydrogen atom or a C₁-C₈ alkyl radical;
(ii) the R₁ and R₂ radicals, which are identical or different, can also denote a residue of following formula (1) : 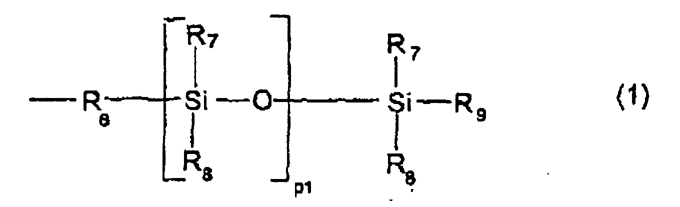 in which:
- R₆ denotes a covalent bond, a linear or branched C₁-C₄ alkyl radical or a residue of formula -C_{m₁}H_{2m₁}-O- where m₁ is a number from 1 to 4;
- p₁ is a number from 0 to 5;
- the R₇, R₈ and R₉ radicals, which are identical or different, denote a C₁-C₁₈ alkyl radical, a C₁-C₁₈ alkoxy radical or a residue of formula: 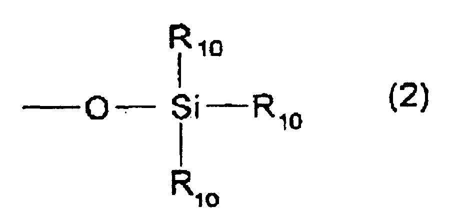 where R₁₀ is a C₁-C₅ alkyl radical;
- A₁ denotes a residue corresponding to one of the following formulae: 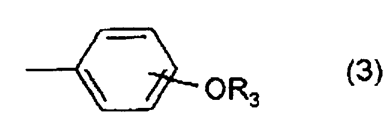 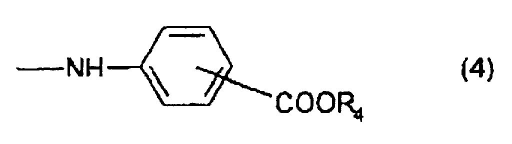 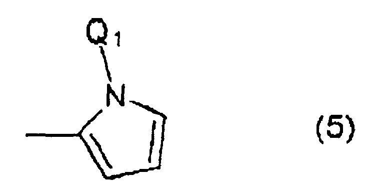
in which:
- R₃ denotes a hydrogen atom, a C₁-C₁₀ alkyl radical, a radical of formula -(CH₂CHR₅-O)_{n₁}R₄ where n₁ is a number from 1 to 16, or a residue of structure -CH₂-CH(OH)-CH₂OT₁ with T₁ having the same meaning indicated above;
- R₄ denotes hydrogen, a metal cation M, a C₁-C₅ alkyl radical or a residue of formula -(CH₂)_{m₂}-OT₁, where m₂ is a number from 1 to 4 and T₁ has the same meaning indicated above;
- Q₁ is a C₁-C₁₈ alkyl radical, the said first and second screening agents being present in a proportion producing a synergic activity with regard to the sun protection factors conferred.

2. Composition according to Claim 1, where the compound of formula (II) is chosen from the group consisting of:
- 2,4-bis{[4-(2-ethylhexyloxy)-2-hydroxy]phenyl}-6-(4-methoxyphenyl)-1,3,5-triazine;
- 2,4-bis{[4-(3-(2-propyloxy)-2-hydroxypropyloxy)-2-hydroxy]phenyl}-6-(4-methoxyphenyl)-1,3,5-triazine;
- 2,4-bis{[4-(2-ethylhexyloxy)-2-hydroxy]phenyl}-6-[4-(2-methoxyethylcarboxyl)phenylamino]-1,3,5-triazine;
- 2,4-bis{[4-tris((trimethylsiloxy)silylpropyloxy)-2-hydroxy]phenyl}-6-(4-methoxyphenyl)-1,3,5-triazine;
- 2,4-bis{[4-(2"-methylpropenyloxy)-2-hydroxy]phenyl}-6-(4-methoxyphenyl)-1,3,5-triazine;
- 2,4-bis{[4-(1',1',1',3',5',5',5'-heptamethyltrisiloxy-2"-methylpropyloxy)-2-hydroxy]phenyl}-6-(4-methoxyphenyl)-1,3,5-triazine;
- 2,4-bis{[4-(3-(2-propyloxy)-2-hydroxypropyloxy]-2-hydroxy]phenyl}-6-[(4-ethylcarboxyl)phenylamino]-1,3,5-triazine;
- 2,4-bis{[4-(2-ethylhexyloxy)-2-hydroxy]phenyl}-6-(1-methylpyrrol-2-yl)-1,3,5-triazine.

3. Composition according to either one of Claims 1 and 2, **characterized in that** the compound of formula (II) is chosen from the group consisting of:
- 2,4-bis{[4-(2-ethylhexyloxy)-2-hydroxy]phenyl}-6-(4-methoxyphenyl)-1,3,5-triazine;
- 2,4-bis{[4-tris((trimethylsiloxy)silylpropyloxy)-2-hydroxy]phenyl}-6-(4-methoxyphenyl)-1,3,5-triazine;
- 2,4-bis{[4-(1',1',1',3',5',5',5'-heptamethyltrisiloxy-2"-methylpropyloxy)-2-hydroxy]phenyl}-6-(4-methoxyphenyl)-1,3,5-triazine.

4. Composition according to any one of Claims 1 to 3, **characterized in that** the concentration of screening agent of the bisresorcinyltriazine derivative type is between 0.1 and 15% by weight with respect to the total weight of the composition.

5. Composition according to Claim 4, **characterized in that** the said concentration is between 0.2 and 10% by weight with respect to the total weight of the composition.

6. Composition according to any one of Claims 1 to 5, **characterized in that** benzene-1,4-di(3-methylidene-10-camphorsulphonic acid) and its salts corresponds to the following formula (I) : 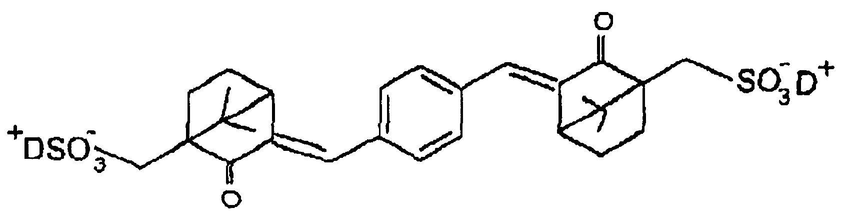 in which D denotes a hydrogen atom, an alkali metal or an NH(R₂₅)₃⁺ radical in which the R₂₅ radicals, which can be identical or different, denote a hydrogen atom, a C₁-C₄ alkyl or hydroxyalkyl radical or an Mⁿ⁺/n group, Mⁿ⁺ denoting a polyvalent metal cation in which n is equal to 2 or 3 or 4, Mⁿ⁺ preferably denoting a metal cation chosen from Ca²⁺, Zn²⁺, Mg²⁺, Ba²⁺, Al³⁺ and Zr⁴⁺.

7. Composition according to any one of Claims 1 to 6, **characterized in that** benzene-1,4-di(3-methylidene-10-camphorsulphonic acid) or one of its salts is generally present at a total concentration of between 0.1 and 15% by weight approximately with respect to the total weight of the composition.

8. Composition according to Claim 7, where the concentration is between 0.2 and 10% by weight approximately with respect to the total weight of the composition.

9. Composition according to any one of Claims 1 to 8, **characterized in that** the said cosmetically acceptable vehicle is provided in the form of an emulsion of oil-in-water type.

10. Composition according to any one of Claims 1 to 9, **characterized in that** it furthermore comprises one or more additional hydrophilic or lipophilic organic screening agents which are active in the UV-A and/or UV-B regions, other than the said first and second screening agents.

11. Composition according to Claim 10, **characterized in that** the said additional organic screening agents are chosen from cinnamate derivatives, salicylic derivatives, camphor derivatives, benzimidazole derivatives, β,β-diphenylacrylate derivatives, triazine derivatives other than those defined in the preceding claims, p-aminobenzoic acid derivatives, or screening polymers and screening silicones.

12. Composition according to any one of Claims 1 to 11, **characterized in that** it furthermore comprises, as additional UV photoprotective agents, pigments or nanopigments formed of metal oxides which are coated or non-coated.

13. Composition according to Claim 12, **characterized in that** the said pigments or nanopigments are chosen from titanium, zinc, iron, zirconium or cerium oxides and their mixtures which are coated or non-coated.

14. Composition according to any one of Claims 1 to 13, **characterized in that** it additionally comprises at least one agent for the artificial tanning and/or browning of the skin.

15. Composition according to any one of Claims 1 to 14, **characterized in that** it additionally comprises at least one adjuvant chosen from fatty substances, organic solvents, thickeners, softeners, antioxidants, opacifiers, stabilizers, emollients, hydroxy acids, antifoaming agents, moisturizing agents, vitamins, fragrances, preservatives, surfactants, fillers, sequestering agents, polymers, propellants, basifying or acidifying agents, or dyes.

16. Composition according to any one of Claims 1 to 15, **characterized in that** it is a composition for the protection of the human epidermis or is an antisun composition and **in that** the composition is provided in the form of a non-ionic vesicular dispersion, of an emulsion, in particular of an emulsion of oil-in-water type, of a cream, of a milk, of a gel, of a cream gel, of a suspension, of a dispersion, of a powder, of a solid tube, of a foam or of a spray.

17. Composition according to any one of Claims 1 to 16, **characterized in that** it is a composition for making up the eyelashes, eyebrows or skin and **in that** the composition is provided in the anhydrous or aqueous, pasty or solid form of an emulsion, of a suspension or of a dispersion.

18. Composition according to any one of Claims 1 to 16, **characterized in that** it is a composition intended for the protection of the hair against ultraviolet rays and **in that** the composition is provided in the form of a shampoo, of a lotion, of a gel, of an emulsion or of a non-ionic vesicular dispersion.

19. Use of the composition defined in any one of the preceding Claims 1 to 16 in the manufacture of cosmetic compositions for the protection of the skin and/or hair against ultraviolet radiation, in particular solar radiation.

20. Cosmetic treatment process for protecting the skin and/or hair against ultraviolet radiation, in particular solar radiation, **characterized in that** it consists in applying, to the skin and/or hair, an effective amount of a composition as defined in any one of Claims 1 to 18.
